(19) Europäisches Patentamt
European Patent Office
Office européen des brevets

(11) Veröffentlichungsnummer: **0 054 142**
**B1**

(12) # EUROPÄISCHE PATENTSCHRIFT

(45) Veröffentlichungstag der Patentschrift: **03.07.85**

(51) Int. Cl.⁴: **C 07 C 143/16,** C 07 C 139/00

(21) Anmeldenummer: **81108703.0**

(22) Anmeldetag: **22.10.81**

(54) **Verfahren zur Herstellung von Vinylsulfonaten.**

(30) Priorität: **13.12.80 DE 3047028**

(43) Veröffentlichungstag der Anmeldung: **23.06.82 Patentblatt 82/25**

(45) Bekanntmachung des Hinweises auf die Patenterteilung: **03.07.85 Patentblatt 85/27**

(84) Benannte Vertragsstaaten: **BE CH DE FR GB LI**

(56) Entgegenhaltungen:
**DE - A - 2 058 776**
**DE - C - 677 843**
**DE - C - 1 277 247**

**Chemical Abstracts Band 52, Nr. 5 10. März 1958 Columbus, Ohio, USA D.S. BRESLOW et al. "Synthesis of sodium ethylenesulfonate from ethylene" Seite 1958, Spalte 2, Abstract Nr. 3662i bis Seite 1959, Spalte 1, Abstract Nr. 3663c**

(73) Patentinhaber: **BASF Aktiengesellschaft, Carl-Bosch-Strasse 38, D-6700 Ludwigshafen (DE)**

(72) Erfinder: **Distler, Harry, Dr., In den Hahndornen 5, D-6719 Bobenheim (DE)**
Erfinder: **Widder, Rudi, Dr., In der Taesch 7, D-6906 Leimen (DE)**
Erfinder: **Krusche, Goerg, Dr., Max-Beckmann-Strasse 1, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein neues Verfahren zur Herstellung von Vinylsulfonaten durch Umsetzung von flüssigem Carbylsulfat zwischen 32 bis 45°C mit wässriger Alkalilauge.

Es ist bekannt, dass man durch Behandlung von Äthionaten mit wässrigen Alkalien zwischen 50 und 80°C Vinylsulfonat herstellen kann (DE-PS 677 843). Es wird ausdrücklich darauf hingewiesen, dass die Gefahr einer Umwandlung des Endstoffs in Isäthionsäure besteht. Daher wird bei tiefer Temperatur, z.B. mit 5n-Natronlauge bei Zimmertemperatur, rasch umgesetzt, hingegen bei Verwendung von 1n-2n-Lauge bei 50°C umgesetzt werden muss. Im Falle von Erdalkali oder Alkalicarbonaten werden Temperaturen von 50° bis 80°C angewendet. In allen Beispielen werden 10- bis 15-gewichtsprozentige Natronlauge oder Barythydrat bei 50° bis 65°C verwendet. Zwar soll auch Carbylsulfat anwendbar sein, in den Beispielen wird aber nur Natriumäthionat veranschaulicht.

Es ist ferner ein Zweistufenprozess bekannt, wobei man Carbylsulfat zunächst bei 25 bis 30°C mit Lauge behandelt, der dann die eigentliche Reaktion bei hoher Temperatur (65 bis 70°C) fogen muss (J. Am. Chem. Soc., Band 79, Seiten 5000-5002 (1957). Die Ausbeuten betragen hier 89% der Theorie.

Mehrfach wird auf die hohe Instabilität des Vinylsulfonates hingewiesen und Vorschläge zur Stabilisierung gemacht (J. Am. Chem. Soc., Band 76, Seiten 5361-5363 (1954). Die erhaltenen Reaktionsprodukte enthalten mehr oder weniger höhere Polymeranteile, welche die Verwendung als Monomer problematisch oder unmöglich machen.

Ähnlich wird ein Zweistufenprozess der Vinylsulfonatherstellung in der DE-OS 2 058 776 beschrieben. Der jeweilige Polymeranteil blieb hier unberücksichtigt. Es wird ausdrücklich darauf aufmerksam gemacht, dass das Carbylsulfat bei Temperaturen über 40°C unbeständig gegen wässrige Alkali ist, bei Zimmertemperatur hingegen «friert» die Umsetzung ein, um dann aber bisweilen explosionsartig unter Bildung von Polysulfonaten und Isäthionat abzulaufen. Es wird daher nach der ersten Stufe bei 30 bis 50° eine zweite Stufe bei 70 bis 150°C für erforderlich gehalten, um eine befriedigende Ausbeute zu erhalten. Man verwendet festes Carbylsulfat.

In der Angewandten Chemie, Band 1965, Seite 292 wird für die Herstellung von Vinylsulfonsäurearylestern durch Umsetzung von Phenolen mit Carbylsulfat in alkalischen Medien eine Temperatur von 0°C empfohlen.

Es wurde nun gefunden, dass man Vinylsulfonate durch Behandlung von Carbylsulfat im basischen Bereich vorteilhaft erhält, wenn man flüssiges Carbylsulfat bei einer Temperatur zwischen 32 und 45°C mit einer wässrigen Alkalilauge umsetzt.

Die Umsetzung lässt sich für den Fall der Verwendung von Natronlauge durch die folgenden Formeln wiedergeben:

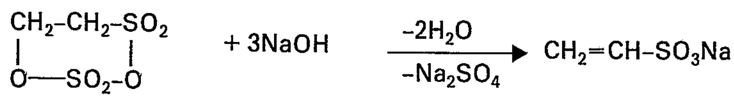


Im Hinblick auf den Stand der Technik liefert das erfindungsgemässe Verfahren in einfacherer und wirtschaftlicherer Weise Vinysulfonate in besserer Ausbeute und Reinheit. Es ist insbesondere angesichts des verwendeten flüssigen Carbylsulfats und des erfindungsgemässen Temperaturbereiches überraschend, dass die Reaktion leicht und mit besten Ergebnissen erfolgt; man hätte erhöhte Polymerisation einerseits und infolge langsamer Reaktion andererseits schlechte Raum-Zeit-Ausbeuten erwartet. Zumindest war die Bildung eines heterogenen Gemisches zu vermuten; die Reinheit des Endstoffes und dadurch eine Einsparung von Reinigungsoperationen des erfindungsgemässen Verfahrens sind überraschend.

Ein entscheidendes Merkmal ist die Umsetzung mit flüssigem Carbylsulfat, das in der Regel ohne zusätzliche Lösungsmittel verwendet wird. Vorteilhaft führt man das Carbylsulfat der wässrigen Lauge so zu, dass das Einlaufsrohr nicht in das Ausgangsgemisch taucht. Dadurch werden Wärmestauungen (Reaktionswärme 400 Kcal pro Mol) und unerwünschte Nebenreaktionen (Polymere, Isäthionatbildung) vermieden.

Als wässrige Alkalilauge sind Natronlauge und Kalilauge bevorzugt. Man verwendet 10- bis 50-, insbesondere 20- bis 25-gewichtprozentige, wässrige Lauge. Man kann die Ausgangsstoffe in stöchiometrischer Menge oder im Überschuss umsetzen, zweckmässig in einem Verhältnis von 3 bis 4, insbesondere 3 bis 3,05 Äquivalenten Hydroxid je Mol Carbylsulfat. Der pH-Wert liegt >7, im allgemeinen von 8-13, insbesondere 9-12.

Die Umsetzung wird bei einer Temperatur zwischen 32 und 45°C, vorteilhaft zwischen 32 und 40°C, insbesondere zwischen 32 bis 37°C, drucklos oder unter Druck, kontinuierlich oder diskontinuierlich durchgeführt.

Die Reaktion kann wie folgt durchgeführt werden: Ein Gemisch von Carbylsulfat und wässriger Lauge wird während 2 bis 8 Stunden bei der Reaktionstemperatur gehalten; beim kontinuierlichen Betrieb beträgt die Verweilzeit 0,5 bis eine Stunde. Dann wird das Gemisch langsam auf eine Temperatur von −3 bis −4°C abgekühlt und der Endstoff in üblicher Weise, z.B. durch Filtration und Destillation, isoliert.

Die durch das erfindungsgemässe Verfahren herstellbaren Vinylsulfonate sind wertvolle Ausgangsstoffe für die Herstellung von Pharma, Farbstoffen, Pflanzenschutzmitteln, Netzmitteln

und Emulgatoren. Bezüglich der Verwendung wird auf die vorgenannte Literatur und Ullmanns Encyklopädie der technischen Chemie, Band 3, Seiten 314–315 verwiesen.

Die in den folgenden Beispielen genannten Teile bedeuten Gewichtsteile.

Beispiel 1

In einem Rührkessel werden stündlich unter Rühren bei 35°C 189 Teile flüssiges Carbylsulfat in 480 Teile 25-gewichtsprozentige, wässrige Natronlauge eingetragen, wobei ein pH-Wert 11–12 eingehalten wird. Man erhält stündlich 669 Teile Reaktionsgemisch. Die Umsetzung ist jetzt beendet; zur Isolierung des Endstoffes wird das Gemisch binnen 6 Stunden auf −3°C abgekühlt und durch Filtration das ausgeschiedene Natriumsulfat-Decahydrat abfiltriert. Man erhält 346 Teile einer fast farblosen 37-gewichtsprozentigen Vinylsulfonatlösung, deren Polymeranteil <1% ist. Der Natriumsulfatgehalt ist 0,5%. Ausbeute an Vinylsulfonat 98% der Theorie; Schmelzpunkt 135–140°C. Isäthionat konnte in deutlicher Menge nicht beobachtet werden.

Vergleichsbeispiel 2 (bei 20–25°C)

In einem Rührkessel werden stündlich unter Rühren und starkem Kühlen bei 20–25°C 189 Teile Carbylsulfat in Brocken in 480 Teile 25-gewichtsprozentige, wässrige Natronlauge eingetragen, wobei ein pH-Wert von 11–12 eingehalten wird. Das Reaktionsgemisch nimmt nach kurzer Zeit durch das ausfallende Natriumsulfatdecahydrat und das sich nur mangelhaft umsetzende feste Carbylsulfat eine breiige Konsistenz an, welches die Kühlung sehr erschwert. Gleichzeitig werden alle Leitungen der Anlage verstopft, was zum Abbruch des Versuchs führt.

Vergleichsbeispiel 3 (60°C)

In einem Rührkessel werden stündlich unter Rühren bei 60°C 189 Teile festes Carbylsulfat (in Brocken) in 480 Teile 25-gewichtsprozentige, wässrige Natronlauge eingetragen, wobei ein pH-Wert von 11–12 eingehalten wird. Man erhält stündlich 669 Teile Reaktionsgemisch. Die Umsetzung ist jetzt beendet. Zur Isolierung des Endstoffes wird das Gemisch binnen 6 Stunden auf −3°C abgekühlt und durch Filtration das ausgeschiedene Natriumsulfatdecahydrat abfiltriert.

Man erhält 340 Teile einer gelblichen Vinylsulfonatlösung (Ausbeute an Vinylsulfonat 75% der Theorie), deren Polymeranteil 5% beträgt; der Isäthionatgehalt beträgt 3% und der Natriumsulfatgehalt ist 0,5%. Durch den hohen Polymeranteil ist das Reaktionsprodukt für Folgereaktionen nicht verwendbar.

**Patentanspruch**

Verfahren zur Herstellung von Vinylsulfonaten durch Behandlung von Carbylsulfat im basischen Bereich, dadurch gekennzeichnet, dass man flüssiges Carbylsulfat bei einer Temperatur zwischen 32 und 45°C mit einer 10- bis 50-gewichtsprozentigen, wässrigen Alkalilauge umsetzt.

**Claim**

A process for the preparation of vinyl sulfonates by treating carbyl sulfate in the alkaline range, wherein liquid carbyl sulfate is reacted at from 32 to 45°C with a 10 to 50% strength by weight aqueous caustic alkali solution.

**Revendication**

Procédé de préparation de vinyl-sulfonates à partir de sulfate de carbyle en milieu basique, caractérisé en ce que l'on fait réagir du sulfate de carbyle liquide à une température comprise entre 32 et 45°C avec une lessive alcaline aqueuse d'une concentration de 10 à 50% en poids.